# EUROPEAN PATENT APPLICATION

(11) **EP 4 261 843 A1**
(43) Date of publication of application: **18.10.2023**
(21) Application number: 22290018.5
(22) Date of filing: 15.04.2022
(51) Int. Cl.: G16H 50/20, G16H 50/30

(54) **METHODS AND SYSTEMS FOR ECHOCARDIOGRAPHY-BASED PREDICTION OF CORONARY ARTERY DISEASE**

(71) Applicant: Koninklijke Philips N.V., 5656 AG Eindhoven (NL)
(72) Inventor: Prabhu, David, 5656 AE Eindhoven (NL); Wehle, Simon, 5656 AE Eindhoven (NL); Gessert, Nils Thorbens, 5656 AE Eindhoven (NL); Liu, Jin, 5656 AE Eindhoven (NL); Oliveira, Lucas, 5656 AE Eindhoven (NL); De Craene, Mathieu, 5656 AE Eindhoven (NL); Olivier, Antoine, 5656 AE Eindhoven (NL); Eslami, Parastou, 5656 AE Eindhoven (NL); Van Den Ham, René, 5656 AE Eindhoven (NL); Sun, Deyu, 5656 AE Eindhoven (NL); Rivero, Jose, 5656 AE Eindhoven (NL); Waechter-Stehle, Irina, 5656 AE Eindhoven (NL)
(74) Representative: Philips Intellectual Property & Standards

(57) **Abstract**

A method (100) for providing an analysis of coronary artery disease (CAD), comprising: (i) receiving (120) patient metadata about the patient; (ii) receiving (130) a temporal sequence of 2D and/or 3D ultrasound images of the patient's heart; (iii) selecting (140), by the CAD prediction system, a plurality of ultrasound images from the temporal sequence; (iv) processing (150), using a trained AI algorithm of the CAD prediction system, the selected plurality of ultrasound images to generate a feature map of the selected plurality of ultrasound images; (v) analyzing (160) the generated feature map and the received patient metadata using a trained algorithm of the CAD prediction system to generate a CAD prediction output; (vi) providing (170), via a user interface of the CAD prediction system, the generated CAD prediction output.

## Description

### FIELD OF THE DISCLOSURE

The present disclosure is directed generally to methods and systems for predicting or detecting coronary artery disease using echocardiography.

### BACKGROUND OF THE INVENTION

Coronary artery disease (CAD) is one of the leading causes of death, and one of the major type of CAD, blockages in main coronary arteries, often leads to regional wall motion abnormalities (RWMA) in certain locations of the heart.

Early detection of coronary artery disease is critical to preventative and treatment measures. However, the gold standard for assessment of coronary artery disease is invasive x-ray angiography, which exposes patients to unnecessary risk especially if there is in fact no disease present. Alternatively, CT angiography is increasingly used as a gold standard. However, the procedure also exposes the patient to x-ray radiation, machine access can be limited, and the procedure can be expensive.

In contrast, ultrasound provides a non-invasive, inexpensive, and easily accessible opportunity for CAD assessment. However, direct imaging of the coronary arteries using echocardiography can be challenging. Thus, assessment of regional wall motion abnormality (RWMA) in echocardiographic analysis is often used as a surrogate for direct coronary artery disease assessment, as the condition is correlated with blockages in the major coronary arteries.

However, challenges exist as assessment of RWMA is usually performed using clinician assessment, which can be very user-dependent based on the skill and experience level of the analyst.

### SUMMARY OF THE DISCLOSURE

Accordingly, there is a continued need for methods and systems for more accurate prediction and/or detection of coronary artery disease. Various embodiments and implementations herein are directed to a coronary artery disease (CAD) prediction method and system configured to provide a likelihood of CAD for a patient. The CAD prediction system receives patient metadata about a patient, and receives a temporal sequence of 2D and/or 3D ultrasound images obtained from an ultrasound analysis of the patient's heart. The system selects a plurality of ultrasound images from the temporal sequence, and processes the images using an artificial intelligence (AI) approach to generate a feature map of the selected plurality of ultrasound images. The system uses a trained AI algorithm to analyze the generated feature map and the received patient metadata to generate a CAD prediction output comprising one or more of: (i) a risk or probability of presently having, or developing in the future, CAD for the patient; (ii) a probability of regional wall motion abnormalities (RWMA) for the patient; (iii) a predicted x-ray and/or CT angiography score for the patient; (iv) a prediction of post-procedural CAD interventional success for the patient; and/or (v) a prediction of patient survival with and/or without intervention. The system then uses a user interface to provide the generated CAD prediction output to a user.

Generally, in one aspect, a method for providing an analysis of coronary artery disease (CAD) for a patient using a CAD prediction system is provided. The method includes: (1) receiving patient metadata about the patient; (2) receiving a temporal sequence of 2D and/or 3D ultrasound images obtained from an ultrasound analysis of the patient's heart; (3) selecting, by the CAD prediction system, a plurality of ultrasound images from the temporal sequence; (4) processing, using a trained algorithm of the CAD prediction system, the selected plurality of ultrasound images to generate a feature map of the selected plurality of ultrasound images; (5) analyzing the generated feature map and the received patient metadata using a trained algorithm of the CAD prediction system to generate a CAD prediction output comprising one or more of: (i) a risk or probability of CAD for the patient; (ii) a probability of regional wall motion abnormalities (RWMA) for the patient; (iii) a predicted x-ray and/or CT angiography score for the patient; (iv) a prediction of post-procedural CAD interventional success for the patient; and/or (v) a prediction of patient survival with and/or without intervention; (6) providing, via a user interface of the CAD prediction system, the generated CAD prediction output.

According to an embodiment, the ultrasound analysis of the patient's heart is a transthoracic exam (TTE) or a transoesophageal exam (TEE), and further wherein the ultrasound analysis of the patient's heart is a stress test or a resting (non-stress) exam.

According to an embodiment, the provided CAD prediction output further comprises one or more of the plurality of ultrasound images.

According to an embodiment, the provided one or more of the plurality of ultrasound images comprises a saliency map.

According to an embodiment, the provided CAD prediction output further comprises a confidence score.

According to an embodiment, the trained algorithm of the CAD prediction system processes the selected plurality of ultrasound images to generate a feature map for the selected plurality of ultrasound images in a spatial direction.

According to an embodiment, the trained algorithm of the CAD prediction system processes the selected plurality of ultrasound images to generate a feature map for the selected plurality of ultrasound images in a temporal dimension.

According to an embodiment, the method further includes the step of administering, based on the provided CAD prediction output, a CAD treatment for the patient.

According to another aspect is a system for providing an analysis of coronary artery disease (CAD) for a patient. The system includes: patient metadata about the patient; a temporal sequence of 2D and/or 3D ultrasound images obtained from an ultrasound analysis of the patient's heart; a trained algorithm configured to analyze a plurality of ultrasound images to generate a feature map of the plurality of ultrasound images; a trained algorithm configured to generate a CAD prediction output; a processor configured to: (i) select a plurality of ultrasound images from the temporal sequence; (ii) process, using the trained algorithm of the CAD prediction system, the selected plurality of ultrasound images to generate a feature map of the selected plurality of ultrasound images; (iii) analyze the generated feature map and the received patient metadata using the trained algorithm of the CAD prediction system to generate a CAD prediction output comprising one or more of: a risk or probability of CAD for the patient; a probability of regional wall motion abnormalities (RWMA) for the patient; a predicted x-ray and/or CT angiography score for the patient; a prediction of post-procedural CAD interventional success for the patient; and/or a prediction of patient survival with and/or without intervention; and a user interface configured to provide the generated CAD prediction output.

According to another aspect is a non-transitory computer readable storage medium having computer readable program code embodied therein for causing a coronary artery disease (CAD) prediction system to provide an analysis of coronary artery disease for a patient, by: receiving patient metadata about the patient; receiving a temporal sequence of 2D and/or 3D ultrasound images obtained from an ultrasound analysis of the patient's heart; selecting a plurality of ultrasound images from the temporal sequence; processing, using a trained AI algorithm of the CAD prediction system, the selected plurality of ultrasound images to generate a feature map of the selected plurality of ultrasound images; analyzing the generated feature map and the received patient metadata using a trained algorithm of the CAD prediction system to generate a CAD prediction output comprising one or more of: (i) a risk or probability of CAD for the patient; (ii) a probability of regional wall motion abnormalities (RWMA) for the patient; (iii) a predicted x-ray and/or CT angiography score for the patient; (iv) a prediction of post-procedural CAD interventional success for the patient; and/or (v) a prediction of patient survival with and/or without intervention; providing, via a user interface of the CAD prediction system, the generated CAD prediction output

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

### BRIEF DESCRIPTION OF THE DRAWINGS

In the drawings, like reference characters generally refer to the same parts throughout the different views. The figures showing features and ways of implementing various embodiments and are not to be construed as being limiting to other possible embodiments falling within the scope of the attached claims. Also, the drawings are not necessarily to scale, emphasis instead generally being placed upon illustrating the principles of the various embodiments.
Fig. 1 is a flowchart of a method for providing provide a likelihood of CAD for a patient, in accordance with an embodiment.
Fig. 2 is a schematic representation of a CAD prediction system, in accordance with an embodiment.
Fig. 3 is a flowchart of a method for providing provide a likelihood of CAD for a patient, in accordance with an embodiment.
Fig. 4 is a flowchart of a method for providing provide a likelihood of CAD for a patient, in accordance with an embodiment.
Fig. 5 is a schematic representation of an output of a CAD prediction system, in accordance with an embodiment.
Fig. 6 is a flowchart of a method for training a machine learning algorithm of a CAD prediction system, in accordance with an embodiment.

### DETAILED DESCRIPTION OF EMBODIMENTS

The present disclosure describes various embodiments of a system and method configured to generate a prediction of coronary artery disease. More generally, Applicant has recognized and appreciated that it would be beneficial to provide improved methods and systems for the non-invasive analysis and prediction of coronary artery disease. Accordingly, a CAD prediction system receives patient metadata about a patient, and receives a temporal sequence of 2D and/or 3D ultrasound images obtained from an ultrasound analysis of the patient's heart. The system selects a plurality of ultrasound images from the temporal sequence, and processes the images using a trained algorithm to generate a feature map of the selected plurality of ultrasound images. The system uses a trained algorithm to analyze the generated feature map and the received patient metadata to generate a CAD prediction output comprising one or more of: (i) a risk or probability of CAD for the patient; (ii) a probability of regional wall motion abnormalities (RWMA) for the patient; (iii) a predicted x-ray and/or CT angiography score for the patient; (iv) a prediction of post-procedural CAD interventional success for the patient; and/or (v) a prediction of patient survival with and/or without intervention. The system then uses a user interface to provide the generated CAD prediction output to a user.

According to an embodiment, therefore, is a novel end-to-end learning pipeline that predicts the findings from x-ray/CT angiography based on 2D/3D rest/stress transthoracic (TTE) or transesophageal (TEE) echocardiography images and patient metadata. One or multiple sequences can be processed by the algorithm at once. The algorithm is robust to different manifestations of input image quality, resolution, vendor specificities, and so on. Moreover, the tool enables the prediction of global or localized RWMA with classification or regression of blockages in the main coronary arteries.

According to an embodiment, the methods and systems utilizes an AI solution for detection of CAD using echocardiography. This approach aims to detect the presence of RWMA in the 2D/3D TTE/TEE echo loops. Next, the systems and methods aim to predict the presence of CAD based on CT angiography images. That is, the systems and methods utilize AI (e.g., 3D and/or 4D convolutional neural networks (CNN)) to predict CT-angiography based assessment of CAD, using the ultrasound images and associated patient metadata (age, weight, ECG features, etc.) as input. The system can use global average pooling within the channels dimension of the CNN to aggregate over the temporal dimension. Or the system can use CNNs with a combination of recurrent units such as RNN to aggregate over the temporal dimension. The algorithm used for CAD/RWMA assessment can be applied on cart or by a cloud service - potentially enabling many more patients access to CAD evaluation. The output is connected to one or multiple classification or prediction layers. The output of the algorithm is more consistent than manual assessments of RWMA. Moreover, ultrasound imaging is safer, cheaper, and more accessible compared to other imaging modalities used to assess CAD. The output of the pipeline can comprise predictions for several observables and saliency maps, indicating decisive regions in the input sequence with respect to the individual observables. Finally, the algorithm can help determine next steps in the clinical pathway by predicting the success/survival rate using intervention/no intervention.

According to an embodiment, the methods and systems can be used to support cardiologists to select/filter patients/studies or sequences within a study for further assessment. The algorithm can also indicate regions of interest within the images showing possible signs of RWMA. In this manner the methods and systems can lead to significant productivity gains in cardiology workflow.

According to an embodiment, the methods and systems result in one or more of the following as output:
1. A classification of either global or local RWMA assessment, where the classification of RWMA cases can either be defined as normal or abnormal. Moreover, the abnormal class can be further stratified into either: hypokinetic, hyperkinetic, or dyskinetic types.
2. A prediction of CT-angiography report findings based on the echocardiography images + patient metadata. According to an embodiment, the output can either be a classification of blockage/noblockage predicted, and/or a regression of the amount of blockage in any coronary artery (e.g. 80% blockage in LAD predicted).
3. A prediction of x-ray-angiography report findings based on the echocardiography images + patient metadata. According to an embodiment, the output can either be a classification of blockage/noblockage predicted, and/or a regression of the amount of blockage in any coronary artery (e.g. 80% blockage in LAD predicted).
4. A prediction of post-procedural CAD interventional success, with markers including likelihood of atherectomy success (shockwave, rotational atherectomy) or likelihood of stent intervention success.
5. A prediction of patient survival with/without intervention, etc. (Cox hazard prediction).

According to an embodiment, some of the existing disadvantages overcome by the methods and systems disclosed or otherwise envisioned herein are:
1. End-to-end learning, removing the need for feature extraction.
2. Use of either 2D/3D resting/stress TTE/TEE ultrasound loops with associated patient metadata (age, weight, ECG features, etc.) as input, with a spatiotemporal deep learning architecture.
3. Cheaper and safer than x-ray/CT angiography, as ultrasound exams are relatively inexpensive in nature, more easily accessible, and do not expose the patients to radiation.
4. A consistent and repeatable identification of RWMA, as opposed to qualitative clinician assessment which can vary among users.
5. A prediction of x-ray/CT angiography outcomes based on echo-images, potentially reducing the number of normal patients who go for these more expensive, invasive, and risky procedures.
5. The ability to track long-term patient progress, starting with earlier assessment of CAD (as opposed to CT or angiography).
6. Easier access to CAD risk assessment for more people using an automated or cloud-based approach.
7. Improving cardiac workflow for experts with pre-assessment of echo images.
8. Prediction of post-procedural CAD interventional success, with markers including likelihood of atherectomy success (shockwave, rotational atherectomy) or likelihood of stent intervention success.
9. Prediction of patient survival with/without intervention, etc. (Cox hazard prediction).

According to an embodiment, the systems and methods described or otherwise envisioned herein can, in some non-limiting embodiments, be implemented as an element for a commercial product for ultrasound imaging or analysis, or as an element for a commercial product for cardiovascular analysis such as Philips^{®} IntelliSpace Cardiovascular (ISCV) (available from Koninklijke Philips NV, the Netherlands), or as an element for a commercial product for patient analysis or monitoring, such as the Philips Patient Flow Capacity Suite (PFCS), or any suitable system.

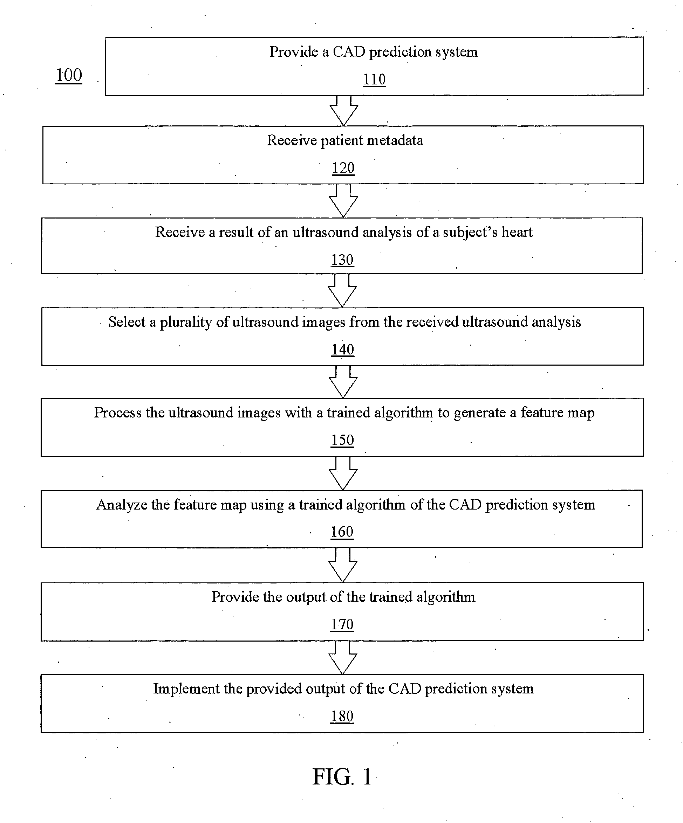

Referring to Fig. 1, in one embodiment is a flowchart of a method 100 for providing a likelihood of coronary heart disease (CAD) for a patient using a CAD prediction system. The methods described in connection with the figures are provided as examples only, and shall be understood to not limit the scope of the disclosure. The CAD prediction system can be any of the systems described or otherwise envisioned herein. The CAD prediction system can be a single system or multiple different systems.

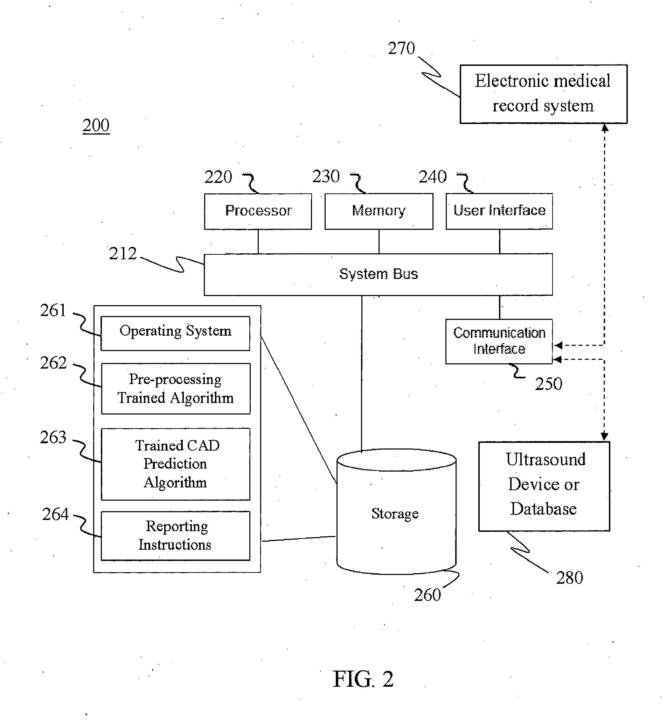

At step 110 of the method, a CAD prediction system 200 is provided. Referring to an embodiment of a CAD prediction system 200 as depicted in Fig. 2, for example, the system comprises one or more of a processor 220, memory 230; user interface 240, communications interface 250, and storage 260, interconnected via one or more system buses 212. It will be understood that Fig. 2 constitutes, in some respects, an abstraction and that the actual organization of the components of the system 200 may be different and more complex than illustrated. Additionally, CAD prediction system 200 can be any of the systems described or otherwise envisioned herein. Other elements and components of patient analysis system 200 are disclosed and/or envisioned elsewhere herein.

At step 120 of the method, the CAD prediction system receives patient metadata about the patient for which an ultrasound exam will be performed. The patient metadata can be any information about the patient that the CAD prediction system can or may utilize for analysis as described or otherwise envisioned herein. According to an embodiment, the patient metadata comprises one or more of demographic information about the patient, inpatient or outpatient status of the patient, medical history of the patient, a diagnosis for the patient, and a reason for the ultrasound to be performed. For example, demographic information may comprise information about the patient such as name, age, body mass index (BMI), and any other demographic information. The inpatient or outpatient status of the patient may comprise any information about whether the patient is admitted or is an outpatient, or any other status of the patient. The medical history of the patient may be any historical admittance or discharge information, historical treatment information, historical diagnosis information, historical exam or imaging information, and/or any other information. The diagnosis for the patient may be any information about a medical diagnosis for the patient, historical and/or current. The reason for the ultrasound to be performed may be any purpose, reason, need, or other impetus for the exam.

The patient metadata is received from one or a plurality of different sources. According to an embodiment, the patient metadata is received from, retrieved from, or otherwise obtained from an electronic medical record (EMR) database or system 270. The EMR database or system may be local or remote. The EMR database or system may be a component of the CAD prediction system, or may be in local and/or remote communication with the CAD prediction system. The received patient metadata may be utilized immediately, or may be stored in local or remote storage for use in further steps of the method.

At step 130 of the method, the CAD prediction system receives a temporal sequence of 2D and/or 3D ultrasound images obtained from an ultrasound analysis of the patient's heart. The ultrasound analysis of the patient's heart may be any ultrasound exam, including but not limited to a transthoracic exam (TTE) or a transesophageal exam (TEE), and can be a stress test or a resting exam. The ultrasound image data is sent to, obtained by, or otherwise received by the system. The ultrasound image comprises a temporal sequence of ultrasound image data such as a video (also referred to as "loop") comprising a plurality of frames, and/or a plurality of videos each comprising a plurality of frames. Ultrasound image data may be obtained for a single region, or may be obtained for a plurality of different zones or regions. The ultrasound image data may be received by the system in real-time, or may be stored in local and/or remote memory and received by the system at a future point.

The ultrasound image data may be obtained using any ultrasound device or system, which may be any device or system suitable to obtain or otherwise receive ultrasound image data of the patient. One or more parameters of the ultrasound device can be set, adjusted, preprogrammed, or otherwise determined by a healthcare professional. The ultrasound device or system may be remote to, local to, or a component of, the CAD prediction system.

At step 140 of the method, the CAD prediction system selects a plurality of ultrasound images from the temporal sequence. The plurality of images may be the entirety of one or more temporal sequences or a portion of a temporal sequence of an ultrasound exam, such as a portion of the exam imaging a particular region or portion of the heart, blood vessels, or other anatomical region. The selection of the plurality of ultrasound images from the temporal sequence can be an automated selection, a selection from a user, or a combination of the two. For example, the CAD prediction system can be designed or programmed to select a certain number of images from a sequence, such as a minimum number of images and/or a certain number of images spanning a minimum amount of exam or imaging time. The selected plurality of ultrasound images from the temporal sequence can be utilized by the CAD prediction system immediately, or can be stored in local and/or remote memory and utilized by the system at a future point.

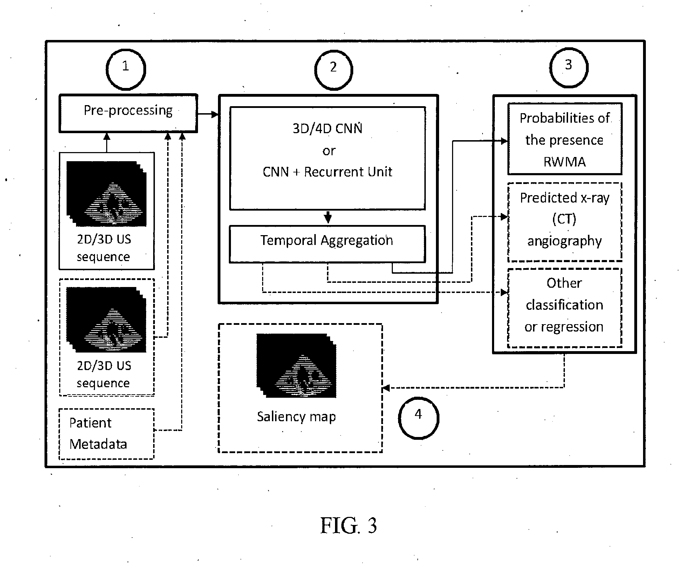

Referring to Fig. 3 is an overview of an embodiment of the methods and systems described or otherwise envisioned herein. Dashed lines indicate possible or optional embodiments. According to an embodiment, the systems and methods comprise: (1) an input layer with pre-processing, (2) a spatial-temporal neural network section with temporal aggregation, (3) neural network classification heads, and (4) decision visualization with saliency maps. Accordingly, the systems and methods can be separated into three different parts: (1) an input interface, where one or several 2D or 3D ultrasound sequences can be selected (e.g. AP4 + AP3 + AP2, etc.); (2) the main image processing algorithm including image pre-processing, neural network inference and temporal aggregation, and finally multiple prediction heads; (3) for various observables to assess the risk of presence of CAD. Finally, there can be a module (4) to visualize the features on the input images that were important for the neural networks with saliency maps.

According to an embodiment, the methods and systems comprise an interface for input. The interface can be or comprise a Digital Imaging and Communications in Medicine (DICOM) interface, other image sources, raw data, or another interface. For example, the interface can be a visual selection of DICOM US sequences in a stand-alone software or integrated into existing hard- or software. In alternative embodiments the methods and systems can be applied directly on a file or files in a directory. The interface may comprise an indication of whether the input image data/patient metadata is within the domain of data used to train the algorithm (i.e., out-of-distribution indication).

According to an embodiment, the selected plurality of images undergoes pre-processing. For example, the methods and systems can be designed and tuned to extract image information from the input data and perform image-processing transformations on the image sequences. This includes spatial and/or temporal image size adjustments and resampling, intensity normalizations, and cropping, among other pre-processing options. The hyperparameters of these processing steps can be chosen so that the neural network part is insensitive to varieties of inputs.

At step 150 of the method, a trained AI algorithm of the CAD prediction system processes the selected plurality of ultrasound images to generate a feature map of the selected plurality of ultrasound images. According to an embodiment, the trained AI algorithm of the CAD prediction system is a convolutional neural network, although many other types of AI algorithms are possible. According to an embodiment, the trained algorithm of the CAD prediction system processes the selected plurality of ultrasound images to generate a feature map of the selected plurality of ultrasound images in a spatial direction, and/or processes the selected plurality of ultrasound images to generate a feature map by temporally aggregating the selected plurality of ultrasound images in a temporal dimension.

According to an embodiment, the CAD prediction system comprises an end-to-end learning pipeline featuring trained convolutional neural networks. These can be 3D or 4D algorithms that learn feature maps of the input ultrasound sequences. Temporal correlations can be accessed in two different embodiments, first, a convolution in the temporal dimension or second, aggregation with a recurrent unit such as an RNN. In the first approach, convolutions are performed in the spatial and temporal dimension; hence in 3D or 4D, depending on the input (2D/3D + time). Temporal aggregation is done in this case by average pooling through the feature maps in the time dimension leaving feature maps as input for the classification heads. In the second approach, convolutions are performed only in spatial dimension, creating an embedding vector containing the (for the neural network) essential information of the input frame. The temporal aggregation is done by feeding these embedding vectors frame by frame into a recurrent neural network. The output of the latter is then again used as input for the classification head neural networks.

According to an embodiment, when compared to other CNN where only images are given as input to a network, spatio-temporal networks aim at combining the spatial patterns within the frames and the temporal dependencies between them. The underlying idea is that that motion, flow, and dynamics carry information about the nature of the sequence at hand. Moreover, in addition to 2D information, the systems and methods also utilize 3D echo loops and patient metadata (e.g. age, gender, etc.), which may contain more predictive information than 2D imaging data alone.

According to an embodiment, the output of step 160 of the method is a feature map that can be analyzed as described or otherwise envisioned herein. The generated feature map can be utilized immediately, or can be stored in local and/or remote memory and utilized by the system at a future point.

At step 160 of the method, a trained algorithm of the CAD prediction system analyzes the generated feature map and the received patient metadata to generate a CAD prediction output. According to an embodiment, the trained algorithm is a neural network head that utilizes as input feature maps created in step 150 of the method. According to an embodiment, these classification heads and the CNN part are trained simultaneously for optimal performance. For training, the system utilizes a database of patients with either 2D/3D Ultrasound Image loops, and the following corresponding metrics for each patient: coronary/CT angiography findings (acquired within +/- 6 months of the echocardiography image acquisition); interventional procedural outcomes (atherectomy, patient survival, etc.). This dataset is used to train the networks to infer the various proposed endpoints from the input sequences.

According to an embodiment, the trained algorithm of the CAD prediction system can analyze the generated feature map and the received patient metadata to generate a CAD prediction output, where the CAD prediction output can be one or more of the following:
1. a risk or probability of CAD for the patient;
2. a probability of regional wall motion abnormalities (RWMA) for the patient;
3. a predicted x-ray and/or CT angiography score for the patient;
4. a prediction of post-procedural CAD interventional success for the patient; and/or
5. a prediction of patient survival with and/or without intervention.

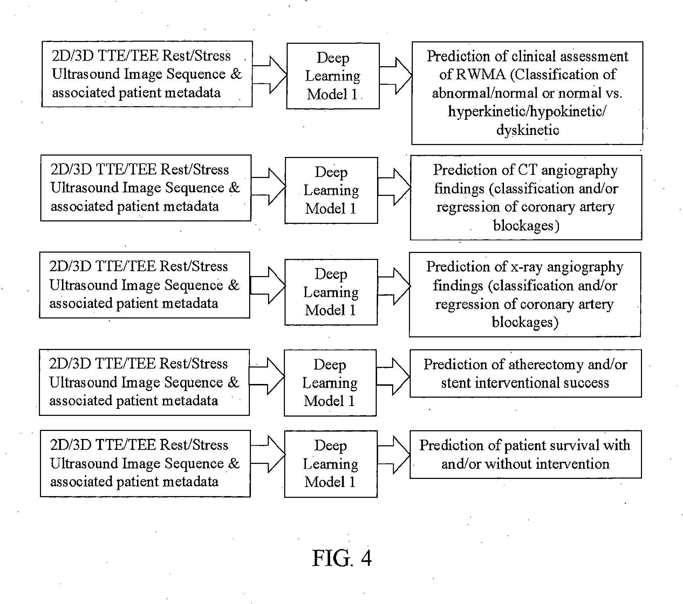

Referring to Fig. 4, in one embodiment, is a flowchart comprising possible embodiments of the trained algorithm of the CAD prediction system at steps 150 and 160 of the method, focusing on specific targets independently. According to an embodiment, the trained algorithm of the CAD prediction system does not need to deliver all the features described herein at the same time. It is also possible that specific versions with a focus on a subset of the described features are developed independently, as shown in Fig. 4. Accordingly, the CAD prediction system can comprise a single trained algorithm that generates and/or processes the feature map created in step 150 of the method to generate one or more of the outputs described or otherwise envisioned herein, or the CAD prediction system can comprise a plurality of trained algorithms to generate one or more of the outputs described or otherwise envisioned herein, with each of the plurality of trained algorithms configured to generate a specific one of the outputs described or otherwise envisioned herein.

At step 170 of the method, the generated CAD prediction output of the one or more trained algorithms is displayed to a medical professional or other user via a user interface of the CAD prediction system. The generated CAD prediction output may be provided to a user via any mechanism for display, visualization, or otherwise providing information via a user interface. According to an embodiment, the information may be communicated by wired and/or wireless communication to a user interface and/or to another device. For example, the system may communicate the information to a mobile phone, computer, laptop, wearable device, and/or any other device configured to allow display and/or other communication of the report. The user interface can be any device or system that allows information to be conveyed and/or received, and may include a display, a mouse, and/or a keyboard for receiving user commands.

According to an embodiment, the display may further comprise one or more of the subject's name, one or more details about the ultrasound analysis, and/or a treatment recommendation, among many other types of information. According to an embodiment, the provided CAD prediction output further comprises one or more of the plurality of ultrasound images that was obtained during the exam and/or utilized during the analysis by the CAD prediction system.

According to an embodiment, the provided one or more of the plurality of ultrasound images comprises a saliency map. Accordingly, the system can generate for each classification head a saliency output that displays on top of the input data regions that were relevant for the individual decision. In this section, the display can also include an indication of classification confidence. Therefore, according to an embodiment, the provided CAD prediction output further comprises a confidence score.

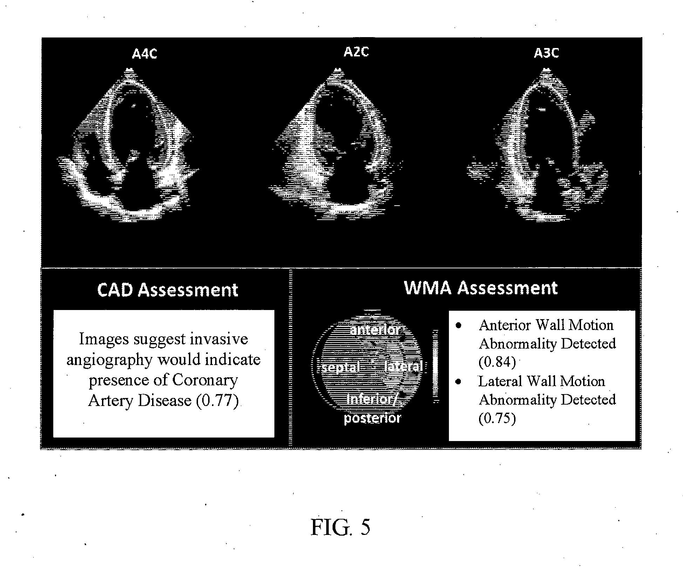

Referring to Fig. 5, in one embodiment, is a possible display of a generated CAD prediction output provided via a user interface of the CAD prediction system. Although not shown, colored regions can be overlayed on top of the original input sequences indicating the saliency intensity with respect to selected observable of the model. The WMA assessment in Fig. 5 includes an anterior wall motion abnormality detection with a confidence score (0.84), and a lateral wall motion abnormality detection with a confidence score (0.75). The display as comprises an overall CAD assessment generated by the one or more trained algorithms of the CAD prediction system, indicating a finding that invasive angiography would likely find the presence of CAD with a likelihood or confidence score of 0.77.

According to an embodiment, the methods and systems described or otherwise envisioned herein focus on the prediction of patient CAD and RWMA. Because this is a cheaper, safer, and more accessible alternative to x-ray/CT-angiography, the system utilizes various outputs from the trained algorithms to perform non-invasive CAD risk stratification of patients. Moreover, longitudinal assessment of patients is also possible to observe whether a patient is **progressing/regressing** with regards to CAD. Therefore, the metrics generated by the methods and systems described or otherwise envisioned herein can be used for the following clinical activities: (i) assessment of therapeutic progression for CAD maintenance; (ii) screening for patients who should undergo a stress echo exam; (iii) screening for patients who require a CT angiography exam; (iv) screening for patient who require x-ray angiography exam; (v) extension to other cardiovascular diseases (e.g. peripheral artery disease); (vi) improved cardiology workflow via faster assessment of RWMA; and/or (vii) reduced inter/intraobserver variability of clinical RWMA assessment. The methods and systems described or otherwise envisioned herein can be used on a variety of systems including premium, point-of-care, value-based, and multi-vendor systems.

At optional step 180 of method 100 depicted in Fig. 1, the generated CAD prediction output provided via a user interface of the CAD prediction system is utilized by a healthcare professional to implement a healthcare treatment for the subject. For example, a clinician or other decisionmaker utilizes the displayed generated CAD prediction output - such as a probability or likelihood of CAD, a probability or likelihood of regional wall motion abnormalities (RWMA) for the patient, a predicted x-ray and/or CT angiography score for the patient, a prediction of post-procedural CAD interventional success for the patient, and/or a prediction of patient survival with and/or without intervention - for patient care decision-making. For example, the probability or likelihood of disease alone or in combination with a prediction or likelihood of CAD interventional success for the patient and/or a prediction of patient survival with and/or without intervention may be utilized by the healthcare professional to initiate, continue, or stop a particular treatment configured to address the patient's CAD. Implementation can comprise a prescription, order, additional testing, and/or another implementation. Many other implementations are possible.

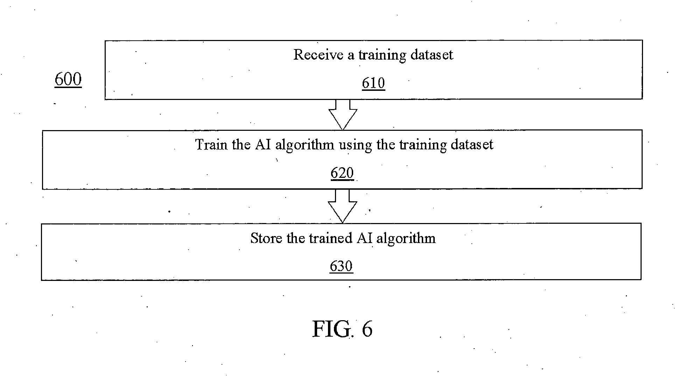

Referring to Fig. 6, in one embodiment, is a flowchart of a method 600 for training the one or more trained algorithm(s) of the CAD prediction system. At step 610 of the method, the system receives a training data set comprising training data about a plurality of patients, such as historical patient data. The training data can comprise input such as ultrasound data for an ultrasound exam for patients with CAD and patients without CAD, clinical diagnoses and outcomes of the patient's treatment, including CAD interventional success for the patient and/or the patient's survival with intervention and without intervention. The training data may be stored in and/or received from one or more databases. The database may be a local and/or remote database. For example, the CAD prediction system may comprise a database of training data.

According to an embodiment, the CAD prediction system may comprise a data pre-processor or similar component or algorithm configured to process the received training data. For example, the data pre-processor analyzes the training data to remove noise, bias, errors, and other potential issues. The data pre-processor may also analyze the input data to remove low-quality data. Many other forms of data pre-processing or data point identification and/or extraction are possible.

At step 620 of the method, the system trains the machine learning algorithm, which will be the algorithm utilized in analyzing the input information as described or otherwise envisioned. The machine learning algorithm is trained using the training data set according to known methods for training a machine learning algorithm. According to an embodiment, the algorithm is trained, using the processed training dataset, to utilize feature maps to generate one or more of the possible CAD outputs described or otherwise envisioned herein, including a confidence, likelihood, or probability score. According to an embodiment, the algorithm is also trained, using the processed training dataset, to generate one or more intervention recommendations based on the determined one or more likelihoods.

At step 630 of the method, the trained model of the CAD prediction system is stored for future use. According to an embodiment, the model may be stored in local or remote storage.

Referring to Fig. 2 is a schematic representation of a CAD prediction system 200. System 200 may be any of the systems described or otherwise envisioned herein, and may comprise any of the components described or otherwise envisioned herein. It will be understood that Fig. 2 constitutes, in some respects, an abstraction and that the actual organization of the components of the system 200 may be different and more complex than illustrated.

According to an embodiment, system 200 comprises a processor 220 capable of executing instructions stored in memory 230 or storage 260 or otherwise processing data to, for example, perform one or more steps of the method. Processor 220 may be formed of one or multiple modules. Processor 220 may take any suitable form, including but not limited to a microprocessor, microcontroller, multiple microcontrollers, circuitry, field programmable gate array (FPGA), application-specific integrated circuit (ASIC), a single processor, or plural processors.

Memory 230 can take any suitable form, including a non-volatile memory and/or RAM. The memory 230 may include various memories such as, for example L1, L2, or L3 cache or system memory. As such, the memory 230 may include static random access memory (SRAM), dynamic RAM (DRAM), flash memory, read only memory (ROM), or other similar memory devices. The memory can store, among other things, an operating system. The RAM is used by the processor for the temporary storage of data. According to an embodiment, an operating system may contain code which, when executed by the processor, controls operation of one or more components of system 200. It will be apparent that, in embodiments where the processor implements one or more of the functions described herein in hardware, the software described as corresponding to such functionality in other embodiments may be omitted.

User interface 240 may include one or more devices for enabling communication with a user. The user interface can be any device or system that allows information to be conveyed and/or received, and may include a display, a mouse, and/or a keyboard for receiving user commands. In some embodiments, user interface 240 may include a command line interface or graphical user interface that may be presented to a remote terminal via communication interface 250. The user interface may be located with one or more other components of the system, or may located remote from the system and in communication via a wired and/or wireless communications network.

Communication interface 250 may include one or more devices for enabling communication with other hardware devices. For example, communication interface 250 may include a network interface card (NIC) configured to communicate according to the Ethernet protocol. Additionally, communication interface 250 may implement a TCP/IP stack for communication according to the TCP/IP protocols. Various alternative or additional hardware or configurations for communication interface 250 will be apparent.

Storage 260 may include one or more machine-readable storage media such as read-only memory (ROM), random-access memory (RAM), magnetic disk storage media, optical storage media, flash-memory devices, or similar storage media. In various embodiments, storage 260 may store instructions for execution by processor 220 or data upon which processor 220 may operate. For example, storage 260 may store an operating system 261 for controlling various operations of system 200.

It will be apparent that various information described as stored in storage 260 may be additionally or alternatively stored in memory 230. In this respect, memory 230 may also be considered to constitute a storage device and storage 260 may be considered a memory. Various other arrangements will be apparent. Further, memory 230 and storage 260 may both be considered to be non-transitory machine-readable media. As used herein, the term non-transitory will be understood to exclude transitory signals but to include all forms of storage, including both volatile and non-volatile memories.

While system 200 is shown as including one of each described component, the various components may be duplicated in various embodiments. For example, processor 220 may include multiple microprocessors that are configured to independently execute the methods described herein or are configured to perform steps or subroutines of the methods described herein such that the multiple processors cooperate to achieve the functionality described herein. Further, where one or more components of system 200 is implemented in a cloud computing system, the various hardware components may belong to separate physical systems. For example, processor 220 may include a first processor in a first server and a second processor in a second server. Many other variations and configurations are possible.

According to an embodiment, the electronic medical record system 270 is an electronic medical records database from which the information about the patient, including clinical information and ultrasound data, may be obtained or received. The electronic medical records database may be a local or remote database and is in direct and/or indirect communication with the CAD prediction system 200. Thus, according to an embodiment, the CAD prediction system comprises an electronic medical record database or system 270.

According to an embodiment, the system comprises one or more ultrasound devices 280 capable of acquiring the required ultrasound images or analysis. According to another embodiment, CAD prediction system 200 is in wired and/or wireless communication with a local or remote ultrasound device 280 capable of acquiring the required ultrasound images or analysis. According to another embodiment, CAD prediction system 200 is in wired and/or wireless communication with a local or remote database 280 which stores the ultrasound images or analysis. The CAD prediction system 200 can obtain the required ultrasound images or analysis from one or more of these sources.

According to an embodiment, storage 260 of system 200 may store one or more algorithms, modules, and/or instructions to carry out one or more functions or steps of the methods described or otherwise envisioned herein. For example, the system may comprise, among other instructions or data, a trained pre-processing algorithm 262, a trained CAD prediction algorithm 264, and/or reporting instructions 264.

According to an embodiment, the trained pre-processing algorithm 262 comprises one or more trained algorithms configured to receive as input a selected plurality of ultrasound images, and configured to generate as output a feature map of the selected plurality of ultrasound images. The pre-processing algorithm 262 can be trained using any known method, including the methods described or otherwise envisioned herein. The AI algorithm 262 can be any type of trainable algorithm, including but not limited to neural networks among many other types. According to an embodiment, the trained convolutional neural network of the CAD prediction system processes the selected plurality of ultrasound images to generate a feature map by convoluting the selected plurality of ultrasound images in a spatial direction. According to another embodiment, the trained convolutional neural network of the CAD prediction system processes the selected plurality of ultrasound images to generate a feature map by temporally aggregating the selected plurality of ultrasound images in a temporal dimension. According to an embodiment, the output of the trained pre-processing algorithm 262 is a feature map that can be analyzed as described or otherwise envisioned herein. The generated feature map can be utilized immediately, or can be stored in local and/or remote memory and utilized by the system at a future point.

According to an embodiment, the trained CAD prediction algorithm 264 comprises one or more trained algorithms configured to receive as input the generated feature map, and configured to generate as output a CAD prediction output as described or otherwise envisioned herein. The CAD prediction algorithm 264 can be trained using any known method, including the methods described or otherwise envisioned herein. The CAD prediction algorithm 264 can be trained using a training data set as described or otherwise envisioned herein. According to an embodiment, the trained algorithm is a neural network head that utilizes as input feature maps. According to an embodiment, these classification heads and the CNN part are trained simultaneously for optimal performance. For training, the system utilizes a database of patients with either 2D/3D ultrasound image loops, and the following corresponding metrics for each patient: coronary/CT angiography findings (acquired within +/- 6 months of the echocardiography image acquisition); interventional procedural outcomes (atherectomy, patient survival, etc.). This dataset is used to train the networks to infer the various proposed endpoints from the input sequences. According to an embodiment, the trained CAD prediction algorithm 264 analyzes the generated feature map and the received patient metadata to generate a CAD prediction output, where the CAD prediction output can be one or more of the following: 1. a risk or probability of CAD for the patient; 2. a probability of regional wall motion abnormalities (RWMA) for the patient; 3. a predicted x-ray and/or CT angiography score for the patient; 4 a prediction of post-procedural CAD interventional success for the patient; and/or 5. a prediction of patient survival with and/or without intervention, among other possible CAD prediction outputs.

According to an embodiment, reporting instructions 264 direct the system to generate and provide to a user via a user interface information comprising the generated CAD prediction output. According to an embodiment, the display may further comprise one or more of the subject's name, one or more details about the ultrasound analysis, and/or a treatment recommendation, among many other types of information. According to an embodiment, the provided CAD prediction output further comprises one or more of the plurality of ultrasound images that was obtained during the exam and/or utilized during the analysis by the CAD prediction system. According to an embodiment, the provided one or more of the plurality of ultrasound images comprises a saliency map. Accordingly, the system can generate for each classification head a saliency output that displays on top of the input data regions that were relevant for the individual decision. In this section, the display can also include an indication of classification confidence. Therefore, according to an embodiment, the provided CAD prediction output further comprises a confidence score. The provided CAD prediction output can further comprise a treatment recommendation, among many other types of information.

The generated CAD prediction output may be provided to a user via any mechanism for display, visualization, or otherwise providing information via a user interface. According to an embodiment, the information may be communicated by wired and/or wireless communication to a user interface and/or to another device. For example, the system may communicate the information to a mobile phone, computer, laptop, wearable device, and/or any other device configured to allow display and/or other communication of the report. The user interface can be any device or system that allows information to be conveyed and/or received, and may include a display, a mouse, and/or a keyboard for receiving user commands.

Accordingly, within the context of the disclosure herein, aspects of the embodiments may take the form of a computer program product embodied in one or more non-transitory computer readable media having computer readable program code embodied thereon. Thus, according to one embodiment is a non-transitory computer-readable storage medium comprising computer program code instructions which, when executed by a processor, enables the processor to carry out a method including: (i) receiving patient metadata about the patient; (ii) receiving a temporal sequence of 2D and/or 3D ultrasound images obtained from an ultrasound analysis of the patient's heart; (iii) selecting a plurality of ultrasound images from the temporal sequence; (iv) processing, using a trained convolutional neural network of the CAD prediction system, the selected plurality of ultrasound images to generate a feature map of the selected plurality of ultrasound images; (v) analyzing the generated feature map and the received patient metadata using a trained algorithm of the CAD prediction system to generate a CAD prediction output comprising one or more of: (a) a risk or probability of CAD for the patient; (b) a probability of regional wall motion abnormalities (RWMA) for the patient; (c) a predicted x-ray and/or CT angiography score for the patient; (d) a prediction of post-procedural CAD interventional success for the patient; and/or (e) a prediction of patient survival with and/or without intervention; (vi) providing, via a user interface of the CAD prediction system, the generated CAD prediction output. The program code may perform entirely on a user's computer, partly on a user's computer, as a stand-alone software package, partly on a user's computer and partly on a remote computer, or entirely on a remote computer or server.

According to an embodiment, the CAD prediction system is configured to process many thousands or millions of datapoints in the input data used to train the system, as well as to process and analyze the received temporal sequence of 2D and/or 3D ultrasound images obtained from an ultrasound analysis of the patient's heart. For example, generating a functional and skilled trained system that comprises both a trained convolutional neural network configured to generate a feature map and a trained algorithm configured to generate a CAD prediction output, requires processing of millions of datapoints from input data to train the algorithms. This requires millions or billions of calculations to generate a novel trained system from those millions of datapoints and millions or billions of calculations. As a result, the trained system is novel and distinct based on the input data and parameters of the machine learning algorithms, and thus improves the functioning of the CAD prediction system. Thus, generating a functional and skilled trained system comprises a process with a volume of calculation and analysis that a human brain cannot accomplish in a lifetime, or multiple lifetimes. By providing an improved non-invasive CAD prediction system, this CAD prediction system has an enormous positive effect on patient analysis, patient diagnosis and prognosis, and patient care compared to prior art systems.

### EXAMPLE

The following is provided as a non-limiting example of one possible embodiment of the methods and systems described or otherwise envisioned herein.

According to an embodiment, a preliminary network was developed to demonstrate performance of this approach, using ~5500 apical 2 chamber (AP2) images. The images either belonged to the category "Anterior RWMA present" (1322 images) or "no Anterior RWMA present" (4139 images). For this example, only non-contrast images were used.

A spatio-temporal 3D CNN was trained on this data with 3-fold patient-stratified cross-validation. Each fold was trained for 15 epochs using Adam optimizer. The model was VGG-inspired architecture with batch normalization. After an initial convolutional layer, there were three processing stages with two convolutional layers each and stride-based downsampling after every stage. This was followed by a global averaging pooling layer and a fully-connected layer with two outputs for the two classes. The entire pipeline was set up within Pytorch.

The present invention may be a system, a method, and/or a computer program product. The computer program product may include a non-transitory computer readable storage medium (or media) having computer readable program instructions thereon for causing a system or processor to carry out aspects of the present invention. The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any combination of the foregoing, among other possibilities. Computer readable program instructions described herein can be downloaded to respective computing/processing devices from a computer readable storage medium or to an external computer or external storage device via a network, for example, the internet, a local area network, and/or a wireless network. Aspects of the present invention are described herein with reference to flowchart illustrations and/or block diagrams of methods, apparatus, systems, and computer program products according to embodiments of the invention. It will be understood that each block of the flowchart illustrations and/or block diagrams, and combinations of blocks in the flowchart illustrations and/or block diagrams, can be implemented by computer readable program instructions.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A method (100) for providing an analysis of coronary artery disease (CAD) for a patient using a CAD prediction system (200), comprising:
receiving (120) patient metadata about the patient;
receiving (130) a temporal sequence of 2D and/or 3D ultrasound images obtained from an ultrasound analysis of the patient's heart;
selecting (140), by the CAD prediction system, a plurality of ultrasound images from the temporal sequence;
processing (150), using a trained AI algorithm of the CAD prediction system, the selected plurality of ultrasound images to generate a feature map of the selected plurality of ultrasound images;
analyzing (160) the generated feature map and the received patient metadata using a trained algorithm of the CAD prediction system to generate a CAD prediction output comprising one or more of: (i) a risk or probability of CAD for the patient; (ii) a probability of regional wall motion abnormalities (RWMA) for the patient; (iii) a predicted x-ray and/or CT angiography score for the patient; (iv) a prediction of post-procedural CAD interventional success for the patient; and/or (v) a prediction of patient survival with and/or without intervention;
providing (170), via a user interface of the CAD prediction system, the generated CAD prediction output.

2. The method of claim 1, wherein the ultrasound analysis of the patient's heart is a transthoracic exam (TTE) or a transoesophageal exam (TEE), and further wherein the ultrasound analysis of the patient's heart is a stress test or a resting (non-stress) exam.

3. The method of claim 1, wherein the provided CAD prediction output further comprises one or more of the plurality of ultrasound images.

4. The method of claim 3, wherein the provided one or more of the plurality of ultrasound images comprises a saliency map.

5. The method of claim 1, wherein the provided CAD prediction output further comprises a confidence score.

6. The method of claim 1, wherein the trained AI algorithm of the CAD prediction system processes the selected plurality of ultrasound images to generate a feature map for the selected plurality of ultrasound images in a spatial direction.

7. The method of claim 1, wherein the trained AI algorithm of the CAD prediction system processes the selected plurality of ultrasound images to generate a feature map by temporally aggregating the selected plurality of ultrasound images in a temporal dimension.

8. The method of claim 1, further comprising the step of administering (180), based on the provided CAD prediction output, a CAD treatment for the patient.

9. A system (200) for providing an analysis of coronary artery disease (CAD) for a patient, comprising:
patient metadata about the patient;
a temporal sequence of 2D and/or 3D ultrasound images obtained from an ultrasound analysis of the patient's heart;
a trained AI algorithm (262) configured to analyze a plurality of ultrasound images to generate a feature map of the plurality of ultrasound images;
a trained algorithm (263) configured to generate a CAD prediction output;
a processor (220) configured to: (i) select a plurality of ultrasound images from the temporal sequence; (ii) process, using the trained AI algorithm of the CAD prediction system, the selected plurality of ultrasound images to generate a feature map of the selected plurality of ultrasound images; (iii) analyze the generated feature map and the received patient metadata using the trained algorithm of the CAD prediction system to generate a CAD prediction output comprising one or more of: a risk or probability of CAD for the patient; a probability of regional wall motion abnormalities (RWMA) for the patient; a predicted x-ray and/or CT angiography score for the patient; a prediction of post-procedural CAD interventional success for the patient; and/or a prediction of patient survival with and/or without intervention; and
a user interface (240) configured to provide the generated CAD prediction output.

10. The system of claim 9, wherein the ultrasound analysis of the patient's heart is a transthoracic exam (TTE) or a transoesophageal exam (TEE), and further wherein the ultrasound analysis of the patient's heart is a stress test or a resting (non-stress) exam.

11. The system of claim 9, wherein the provided CAD prediction output further comprises one or more of the plurality of ultrasound images.

12. The system of claim 9, wherein the provided one or more of the plurality of ultrasound images comprises a saliency map.

13. The system of claim 9, wherein the provided CAD prediction output further comprises a confidence score.

14. The system of claim 9, wherein the trained AI algorithm of the CAD prediction system processes the selected plurality of ultrasound images to generate a feature map for the selected plurality of ultrasound images in a spatial direction, and/or processes the selected plurality of ultrasound images to generate a feature map by temporally aggregating the selected plurality of ultrasound images in a temporal dimension.

15. A non-transitory computer readable storage medium (260) having computer readable program code embodied therein for causing a coronary artery disease (CAD) prediction system (200) to provide an analysis of coronary artery disease for a patient, by:
receiving patient metadata about the patient;
receiving a temporal sequence of 2D and/or 3D ultrasound images obtained from an ultrasound analysis of the patient's heart;
selecting a plurality of ultrasound images from the temporal sequence;
processing, using a trained AI algorithm of the CAD prediction system, the selected plurality of ultrasound images to generate a feature map of the selected plurality of ultrasound images;
analyzing the generated feature map and the received patient metadata using a trained algorithm of the CAD prediction system to generate a CAD prediction output comprising one or more of: (i) a risk or probability of CAD for the patient; (ii) a probability of regional wall motion abnormalities (RWMA) for the patient; (iii) a predicted x-ray and/or CT angiography score for the patient; (iv) a prediction of post-procedural CAD interventional success for the patient; and/or (v) a prediction of patient survival with and/or without intervention;
providing, via a user interface of the CAD prediction system, the generated CAD prediction output.
